# EUROPÄISCHE PATENTSCHRIFT

(11) **EP 0 574 725 B1**
(45) Veröffentlichungstag und Bekanntmachung des Hinweises auf die Patenterteilung: **02.01.1997**
(21) Anmeldenummer: 93108230.9
(22) Anmeldetag: 21.05.1993
(51) Int. Cl.: C07C 67/10, C07C 69/63

(54) **Verfahren zur Herstellung von Halogenessigsäurevinylestern**
Process for the preparation of vinyl esters of halogenacetic acid
Procédé de préparation d'esters vinyliques d'acide halogénoacétique

(30) Priorität: 19.06.1992 DE 4219997
(43) Veröffentlichungstag der Anmeldung: 22.12.1993
(73) Patentinhaber: HOECHST AKTIENGESELLSCHAFT, 65926 Frankfurt am Main (DE)
(72) Erfinder: Seidel, Andreas, Dr., W-5000 Köln (DE); Jägers, Erhard, Dr., W-5303 Bornheim (DE); Bylsma, Friedhelm, W-5042 Erftstadt (DE)

(56) Entgegenhaltungen:
- EP-A- 0 376 075
- DE-A- 2 823 660
- GB-A- 1 486 443
- US-A- 3 188 319

## Beschreibung

Die vorliegende Erfindung betrifft ein Verfahren zur Herstellung von Halogenessigsäurevinylestern der allgemeinen Formel I

HalₙHₘC-COO-CH=CH₂ (I)

worin Hal gleich Cl oder Br, n gleich 1, 2 oder 3, m gleich 3-n ist in flüssiger Phase in Gegenwart einer Palladium(II)-Verbindung als Katalysator, einer Kupfer(II)-Verbindung als Co-Katalysator und einer Natrium-Verbindung als Promotor.

Insbesondere betrifft die vorliegende Erfindung ein Verfahren zur Herstellung von Chloressigsäurevinylester (Cl-CH₂-COC-CH=CH₂).

Copolymere aus Chloressigsäurevinylester und anderen Olefinen, insbesondere Acrylsäure, können räumlich vernetzt werden, wodurch das Polymere neue Eigenschaften erhält. Weiter dient Chloressigsäurevinylester zur Herstellung von Spezialpolymeren für Antifouling-Beschichtungen und schwerentflammbaren Kunststoffen.

Chloressigsäurevinylester kann mittels einer durch Quecksilber-(II)salz katalysierten Vinylierung von Chloressigsäure mit Acetylen in flüssiger Phase hergestellt werden. Das im DE-PS 271 381 beschriebene Verfahren ist jedoch aufgrund der Aufbereitungs- und Umweltprobleme, die aus der Bildung und Abscheidung von katalytisch nicht mehr aktivem elementarem Quecksilber und Quecksilber(I)verbindungen in Nebenreaktionen resultieren, für eine Produktion in technischem Maßstab aus Umweltschutzgründen nicht mehr möglich.

Die EP-A 0 376 075 beschreibt ein Verfahren zur Herstellung von Vinylestern durch Umesterung zwischen einer Carbonsäure und einem Alkenylester in Gegenwart eines Katalysatorsystems, des Palladium(II)-, Kupfer(II)- und Lithiumverbindungen enthält. Als weitere (organische) Edukte werden nach diesem Verfahren nicht-halogenhaltige organische Verbindungen, insbesondere Carbonsäuren und Carbonsäureester verwendet. Die Herstellung von α-Halogencarbonsäuren ist in dieser Schrift nicht erwähnt.

Die DE-A 2 823 660 beschreibt ein Verfahren zur Herstellung durch Umesterung in Gegenwart eines aus Palladium-, Kupfer- und Alkalimetallverbindungen bestehenden Katalysatorsystems, bei dem jedoch keine Katalysatorabtrennung erfolgt.

Ein Verfahren zur Herstellung unhalogenierter Carbonsäurevinylester (3) ist die Umvinylierung eines Carbonsäurevinylesters mit einer Carbonsäure gemäß der Reaktionsgleichung 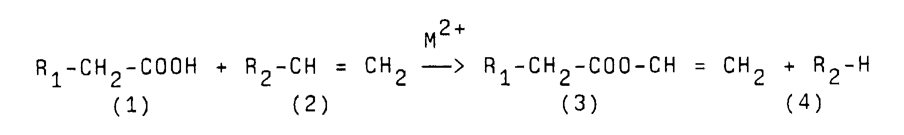 mit R₁ gleich Alkyl oder Aryl und R₂ gleich Carboxylat. Als Katalysator der Umvinylierung können Quecksilber(II)-Verbindungen eingesetzt werden (M = Hg), die jedoch wie bei der Vinylierung mit Acetylen zu Toxitätsproblemen führen.

In der DE-PS 1 127 888 ist ein Verfahren zur Herstellung von Vinylestern höherer unhalogenierter Carbonsäuren beschrieben, bei dem anstelle von Quecksilber(II)-Verbindungen Palladium-Verbindungen als Katalysator eingesetzt werden (M = Pd).

Aus einer von den gleichen Autoren publizierten Schrift (in "Chemische Berichte", Band 102 (1969) Seiten 2939 bis 2950) und unseren Experimenten geht hervor, daß die Palladium(II)-Verbindung während derartiger Umvinylierungen zu metallischem Palladium reduziert wird. Eine kontinuierliche Arbeitsweise ist damit ausgeschlossen.

Bei einer Übertragung dieser Arbeitsweise auf Halogenessigsäuren ist zudem die Bildung großer Nebenproduktmengen zu beobachten, deren spätere Abtrennung von dem Halogenessigsäurevinylester nicht möglich ist.

Es war Aufgabe der vorliegenden Erfindung, ein Verfahren zur Herstellung von Haiogenessigsäurevinylestern zur Verfügung zu stellen, bei dem der abgetrennte Katalysator wiederverwendet werden kann.

Gelöst wird diese Aufgabe durch ein Verfahren zur Herstellung von Halogenessigsäurevinylestern der eingangs beschriebenen Art, dadurch gekennzeichnet, daß man eine Halogenessigsäure der allgemeinen Formel II

HalₙHₘC-COOH (II)

worin Hal, n und m oben genannte Bedeutung haben, mit einem Vinylester der allgemeinen Formel III

R-CH₂-COO-CH=CH₂ (III)

worin R Wasserstoff oder einen Alkylrest mit 1 bis 6 C-Atomen bedeuten, bei 20 bis 100 °C über einen Zeitraum von 0,1 bis 10 Stunden reagieren läßt, danach aus dem Reaktionsgemisch in einer Blitzverdampfung die verdampfbaren Anteile von den Katalysatorkomponenten abtrennt, die abgetrennten Katalysatorkomponenten mit Luftsauerstoff oder Wasserstoffperoxid behandelt, die verdampften Anteile kondensiert und das Kondensat mittels einer Destillation in nicht-umgesetzten Vinylester, rohen Halogenessigsäurevinylester und nicht-umgesetzte Halogenessigsäure trennt, den rohen Halogenessigsäurevinylester bei 5 bis 20 °C mit Wasser extrahiert, wodurch die aus dem eingesetzten Vinylester gebildete Carbonsäure in die wäßrige Phase überführt wird, und den als organische Phase abgetrennten Halogenessigsäurevinylester gegebenenfalls mit einem Trockenmittel behandelt.

Darüber hinaus kann das Verfahren der Erfindung wahlweise und bevorzugt dadurch gekennzeichnet sein, daß man
I.) als Halogenessigsäure Chloressigsäure (Cl-CH₂-COOH) einsetzt;
II.) als Vinylester Vinylacetat (CH₃-COO-CH=CH₂) einsetzt;
III.) als Katalysator PdCl₂, Pd(CH₃-COO)₂ oder Pd(Cl-CH₂-COO)₂ in einer Menge von 10 bis 100 mmol/l flüssiger Phase einsetzt;
IV.) als Co-Katalysator CuCl₂, Cu(CH₃-COO)₂ oder Cu(Cl-CH₂-COO)₂ in einem molaren Verhältnis von 1:3 bis 3:1, bezogen auf das Palladium, einsetzt;
V.) als Promotor NaCl, CH₃-COONa oder Cl-CH₂-COONa in einem molaren Verhältnis von 2:1 bis 10:1, bezogen auf das Palladium, einsetzt;
VI.) eine Vinylester/Halogenessigsäure-Mischung von 60:40 bis 90:10 Gewichtsteile umsetzt;
VII.) den rohen Halogenessigsäurevinylester mit der 1- bis 10-fachen Wassermenge extrahiert;
VIII.)die Extraktion kontinuierlich in einer Mixer-Settler-Apparatur oder einer Kolonne durchführt;
IX.) die Blitzverdampfung in einem Dünnschichtverdampfer durchführt;
X.)
   a) aus dem Kondensat der Blitzverdampfung unter Normaldruck den nicht umgesetzten Vinylester abtrennt, der in dieser Form in das Verfahren rückgeführt werden kann,
   b) den Sumpf der Normaldruckdestillation in einer fraktionierenden Vakuumdestillation in rohen Halogenessigsäurevinylester und gebildete Carbonsäure trennt und
   c) die als Sumpfprodukt bei der Vakuumdestillation verbleibende nicht-umgesetzte Halogenessigsäure in das Verfahren rückführt.
XI. den wasserhaltigen Halogenessigsäurevinylester mit Natriumsulfat, Magnesiumsulfat oder Calciumchlorid als Trockenmittel behandelt.

Beim erfindungsgemäßen Verfahren wird mit einem Überschuß an Vinylester, bezogen auf die eingesetzte Halogenessigsäure, gearbeitet. Dadurch wird einerseits das chemische Gleichgewicht auf die Seite des angestrebten Halogenessigsäurevinylesters verschoben, andererseits wirkt der Vinylester als Lösemittel, wodurch die Zugabe eines anderen Lösemittels überflüssig wird.

Das Verfahren kann drucklos im offenen System oder unter dem sich je nach verwendetem Vinylester einstellenden Dampfdruck im geschlossenen System bei Temperaturen zwischen 20 und 100 °C durchgeführt werden. Die Reaktion ist eine Gleichgewichtsreaktion, deren zeitabhängiger Umsatz zunächst linear, dann aber abflachend einem Grenzwert entgegenstrebt. Um eine Entstehung von hochsiedenden Nebenprodukten zu vermeiden, bricht man die Umsetzung schon am Ende des linearen Umsatzverlaufs vor Erreichen des Gleichgewichts ab. Nebenprodukte sind Acylate, die von halogenierten Essigsäuren und Vinylestern bei höheren Temperaturen und längeren Verweilzeiten gebildet werden.

Die Blitzdestillation kann auch in Form einer Flash-Verdampfung durchgeführt werden, d.h. die heiße Reaktionslösung wird durch Anlegen von Vakuum entspannt. Die Verdampfung muß mit kurzer Verweilzeit und hoher Wärmezufuhr erfolgen, um eine Rückreaktion zu verhindern.

Bei der Reinigung des rohen Halogenessigsäurevinylesters durch die Wasserextraktion sind höhere Temperaturen zu vermeiden, da bei höheren Temperaturen im Kontakt mit Wasser auch Carbonsäuren eine Hydrolyse von Halogenessigsäurevinylester zu Acetaldehyd und Halogenessigsäure katalysieren. Da die Löslichkeit des Esters in der wäßrigen Phase mit deren Carbonsäuregehalt steigt, ist es zur Vermeidung von Produktverlusten zweckmäßig, mehr Wasser in die Extraktion einzusetzen, als zur Ausbildung zweier Phasen erforderlich wäre. Die Extraktion wird vorteilhaft mehrstufig durchgeführt. Ein befriedigendes Ergebnis kann mit einer einstufigen Extraktion in einer Mixer-Settler Apparatur oder durch einfaches Ausschütteln erreicht werden. Der als schwerere organische Phase abgetrennte Halogenessigsäurevinylester enthält 1-4 Gew-% Wasser, welches durch Zusatz eines Trockenmittels entfernt werden kann.

Das Verfahren gemäß der Erfindung kann kontinuierlich oder chargenweise durchgeführt werden.

### Beispiel 1

In einem 1 l mantelbeheizten Glasrührreaktor mit Rückflußkühler werden 3,55 g Palladiumdichlorid (20 mmol) zusammen mit 11,7 g Chloressigsäurenatriumsalz (100 mmol), 5,3 g Kupferdichlorid und 283,5 g (3 mol) Chloressigsäure eingewogen und durch Zugabe von 750 g Vinylacetat (8,72 mol) unter Rühren gelöst. Die grüne Lösung wird auf 50°C erhitzt und bei dieser Temperatur gerührt. Nach zwei Stunden enthält die Lösung 20,2 Gew-% Chloressigsäurevinylester und die Reaktion wird abgebrochen. Anschließend wird die Lösung kontinuierlich aus dem Glasrührreaktor in einer Blitzverdampfung bei 150°C und 150 mbar destilliert. Als Sumpf verbleiben 26,9 g Rückstand, der aus den Katalysatorkomponenten, 1,0 g hochsiedenden Nebenprodukten - im wesentlichen 1-Acetoxy-1-chloracetoxyethan - und Chloressigsäure besteht. Die 1027,5 g Kondensat werden in einer Glockenbodenkolonne (30 mm ⌀) mit 6 Böden unter reduziertem Druck destilliert. Zwischen Kopftemperaturen von 34° bis 56°C bei 220 mbar bis 90 mbar nimmt man 582,8 g einer Fraktion ab, die aus 95,0 Gew-% Vinylacetat, 2,5 Gew-% Acetaldehyd, 1,5 Gew-% Essigsäure und 0,6 Gew-% Chloressigsäurevinylester besteht. Dann werden bei einer Kopftemperatur von 30°C und 15 mbar Druck 334,7 g einer Fraktion abgezogen, welche aus 62,6 Gew-% Chloressigsäurevinylester, 33,4 Gew-% Essigsäure, 2,4 Gew-% Vinylacetat und 0,6 Gew-% Acetaldehyd besteht. Als Destillationssumpf verbleiben 110 g Chloressigsäure. Die aus Chloressigsäurevinylester und Essigsäure bestehende Fraktion wird auf 5°C gekühlt und mit 400 g Wasser von gleicher Temperatur ausgeschüttelt. Die abgetrennte untere Phase wiegt 216,6 g und enthält 95,0 Gew-% Chloressigsäurevinylester und 1,1 Gew-% Wasser.

### Beispiel 2

In einen 100 ml Edelstahlautoklaven mit Glaseinsatz werden 28,35 g Chloressigsäure (0,3 mol), 0,36 g Palladiumdichlorid (2 mmol), 1,17 g Chloressigsäurenatriumsalz (10 mmol) und 0,54 g Kupferdichlorid (4 mmol) eingewogen und in 75,09 g Vinylacetat (0,87 mol) gelöst. Das Gemisch wird auf 70°C aufgeheizt, wobei der Innendruck auf 0,5 bar ansteigt. Nach einer halben Stunde wird auf Raumtemperatur abgekühlt und dem Autoklaven 105,5 g Reaktionsmischung mit den in der nachfolgenden Tabelle angegebenen Gehalten an Acetaldehyd (AcH), Vinylacetat (VINA), Essigsäure (AcOH), Chloressigsäurevinylester (CESVE) und Chloressigsäure (CES) entnommen. Die Reaktionsmischung wird, wie im Beispiel 1 beschrieben, in der Blitzverdampfung in ein Destillat und Katalysatorbestandteile getrennt. Die Katalysatorbestandteile werden mit 1 ml 30 %igem Wasserstoffperoxid (10 mmol) versetzt und durch Erhitzen das Wasser entfernt. Diese regenerierten Katalysatorbestandteile werden sodann mit frisch zugesetzter Chloressigsäure und Vinylacetat erneut umgesetzt. Die Versuchsergebnisse sind in der Tabelle 1 zusammengefaßt.

**Tabelle 1**

| | **Ansatz 1** | **Ansatz 2** | **Ansatz 3** |
|---|---|---|---|
| **Reaktionsgemisch** | **Gew.%** | **Gew.%** | **Gew.%** |
| | | | |
| AcH | 0,9 | 0,8 | 0,6 |
| VINA | 59,5 | 60,7 | 62,4 |
| AcOH | 8,1 | 7,3 | 6,1 |
| CESVE | 13,8 | 12,6 | 10,6 |
| CES | 16,1 | 17,0 | 18,6 |

| **Leistung** | | | |
|---|---|---|---|
| | | | |
| VINA-Umsatz (%) | 16,3 | 14,7 | 12,3 |
| CES-Umsatz (%) | 40,3 | 36,7 | 31,3 |
| RZA (g/l.h) | 290 | 266 | 242 |
| RZA: Raumzeitausbeute | | | |

Die Analysenergebnisse wurden gaschromatografisch ermittelt.

### Beispiel 3 (Vergleich)

Es wurde die Abnahme der Raumzeitausbeute mit Palladium-Katalysator ohne Kupferzusatz ermittelt.

In einen 100 ml Edelstahlautoklaven mit Glaseinsatz werden 28,35 g Chloressigsäure (0,3 mol), 0,36 g Palladiumdichlorid (2mmol) und 1,17 g Chloressigsäurenatriumsalz (10 mmol) eingewogen und in 75,09 g Vinylacetat (0,87 mol) gelöst. Das Gemisch wird auf 70°C aufgeheizt. Nach einer halben Stunde wird auf Raumtemperatur abgekühlt und dem Autoklav 105,0 g Reaktionsgemisch mit den in der nachfolgenden Tabelle 2 angegebenen Gehalten entnommen. Die Reaktionsmischung wird durch eine Blitzverdampfung in ein Destillat und Katalysatorbestandteile getrennt. Die Katalysatorbestandteile werden mit frisch zugesetzter Chloressigsäure und Vinylacetat erneut umgesetzt.

**Tabelle 2**

| | **Ansatz 1** | **Ansatz 2** | **Ansatz 3** |
|---|---|---|---|
| **Reaktionsgemisch** | **Gew.%** | **Gew.%** | **Gew.%** |
| | | | |
| AcH | 0,7 | 0,2 | 0,1 |
| VINA | 60,1 | 70,1 | 71,1 |
| AcOH | 7,8 | 0,5 | > 0,1 |
| CESVE | 13,7 | 1,0 | > 0,1 |
| CES | 16,3 | 26,2 | 26,9 |

| **Leistung** | | | |
|---|---|---|---|
| | | | |
| VINA-Umsatz (%) | 15,6 | 1,6 | 0,2 |
| CES-Umsatz (%) | 39,7 | 3,0 | 0,3 |
| RZA (g/l.h) | 273 | 20 | > 2 |
| RZA: Raumzeitausbeute | | | |

## Patentansprüche

1. Verfahren zur Herstellung von Halogenessigsäurevinylestern der allgemeinen Formel (I)
HalₙHₘC-COO-CH=CH₂ (I)
worin
Hal gleich Cl oder Br,
n gleich 1, 2 oder 3,
m 3-n ist,
in flüssiger Phase in Gegenwart einer Palladium(II)-Verbindung als Katalysator, einer Kupfer(II)-Verbindung als Co-Katalysator und einer Natrium-Verbindung als Promotor, dadurch gekennzeichnet, daß man eine Halogenessigsäure der allgemeinen Formel (II)
HalₙHₘC-COOH (II)
worin Hal, n und m oben genannte Bedeutung haben, mit einem Vinylester der allgemeinen Formel (III)
R-CH₂-COO-CH=CH₂ (III)
worin R Wasserstoff oder einen Alkylrest mit 1 bis 6 C-Atomen bedeuten,
bei 20 °C bis 100 °C über einen Zeitraum von 0,1 bis 10 Stunden reagieren läßt, danach aus dem Reaktionsgemisch in einer Blitzverdampfung die verdampfbaren Anteile von den Katalysatorkomponenten abtrennt,
die abgetrennten Katalysatorkomponenten mit Luftsauerstoff oder Wasserstoffperoxid behandelt,
die verdampften Anteile kondensiert und das Kondensat mittels einer Destillation in nicht umgesetzten Vinylester, rohen Halogenessigsäurevinylester und nicht umgesetzte Halogenessigsäure trennt, den rohen Halogenessigsäurevinylester bei 5 °C bis 20 °C mit Wasser extrahiert, wodurch die aus dem eingesetzten Vinylester gebildete Carbonsäure in die wäßrige Phase überführt wird, und den als organische Phase abgetrennten Halogenessigsäurevinylester gegebenenfalls mit einem Trockenmittel behandelt.

2. Verfahren nach Anspruch 1, dadurch gekennzeichnet, daß man als Halogenessigsäure Chloressigsäure (Cl-CH₂-COOH) einsetzt.

3. Verfahren nach Anspruch 1 oder 2, dadurch gekennzeichnet, daß man als Vinylester Vinylacetat (CH₃-COO-CH=CH₂) einsetzt.

4. Verfahren nach einem der Ansprüche 1 bis 4, dadurch gekennzeichnet, daß man als Katalysator PdCl₂, Pd(CH₃COO)₂ oder Pd(Cl-CH₂-COO)₂ in einer Menge von 10 bis 100 mmol/l flüssiger Phase einsetzt.

5. Verfahren nach einem der Ansprüche 1 bis 4, dadurch gekennzeichnet, daß man als Co-Katalysator CuCl₂, CU(CH₃-COO)₂ oder Cu(Cl-CH₂-COO)₂ in einem molaren Verhältnis von 1:3 bis 3:1, bezogen aufdas Palladium, einsetzt.

6. Verfahren nach einem der Ansprüche 1 bis 5, dadurch gekennzeichnet, daß man als Promotor NaCl, CH₃-COONa oder Cl-CH₂-COONa in einem molaren Verhältnis von 2:1 bis 10:1, bezogen auf das Palladium, einsetzt.

7. Verfahren nach einem der Ansprüche 1 bis 6, dadurch gekennzeichnet, daß man eine Vinylester/Halogenessigsäure-Mischung von 60:40 bis 90:10 Gewichtsteile umsetzt.

8. Verfahren nach einem der Ansprüche 1 bis 7, dadurch gekennzeichnet, daß man den rohen Halogenessigsäurevinylester mit der 1- bis 10fachen Wassermenge extrahiert.

9. Verfahren nach Ansprüch 8, dadurch gekennzeichnet, daß man die Extraktion kontinuierlich in einer Mixer-Settler-Apparatur oder einer Kolonne durchführt.

10. Verfahren nach einem der Ansprüche 1 bis 7, dadurch gekennzeichnet, daß man die Blitzverdampfung in einem Dünnschichtverdampfer durchführt.

11. Verfahren nach einem der Ansprüche 1 bis 10, dadurch gekennzeichnet, daß man
a) aus dem Kondensat der Blitzverdampfung unter Normaldruck den nicht umgesetzten Vinylester abtrennt, der in dieser Form in das Verfahren rückgeführt werden kann,
b) den Sumpf der Normaldruckdestillation in einer fraktionierenden Vakuumdestillation in rohen Hallogenessigsäurevinylester und gebildete Carbonsäure trennt und
c) die als Sumpfprodukt bei der Vakuumdestillation verbleibende nicht umgesetzte Halogenessigsäure in das Verfahren rückführt.

12. Verfahren nach einem der Ansprüche 1 bis 11, dadurch gekennzeichnet, daß man den wasserhaltigen Halogenessigsäurevinylester mit Natriumsulfat, Magnesiumsulfat oder Cacliumchlorid als Trockenmittel behandelt.

## Revendications

1. Procédé de préparation d'halogénoacétates de vinyle de formule générale (I)
HalₙHₘC-COO-CH=CH₂ (I)
dans laquelle
Hal représente Cl ou Br,
n est égal à 1, 2 ou 3,
m est égal à 3-n,
en phase liquide, en présence d'un dérivé du palladium-II qui sert de catalyseur, d'un dérivé du cuivre-II qui sert de catalyseur auxiliaire et d'un dérivé du sodium qui sert d'activateur,
caractérisé en ce que l'on fait réagir un acide halogénoacétique de formule générale II
HalₙHₘC-COOH (II)
dans laquelle Hal, n et m ont les significations indiquées ci-dessus, avec un ester vinylique de formule générale III
R-CH₂-COO-CH=CH₂ (III)
dans laquelle R représente l'hydrogène ou un groupe alkyle en C₁-C₆,
à des températures de 20 à 100°C et pendant une durée de 0,1 à 10 heures, on sépare ensuite du mélange, dans une vaporisation instantanée, les fractions vaporisables des composants du catalyseur,
on traite les composants du catalyseur qui ont été séparés par l'oxygène de l'air ou le peroxyde d'hydrogène,
on condense les fractions vaporisées et on sépare le condensat par distillation en l'ester vinylique non converti, l'halogénoacétate de vinyle brut et l'acide halogénoacétique non converti, on extrait l'halogénoacétate de vinyle brut par l'eau à des températures de 5 à 20°C, ce qui fait passer dans la phase aqueuse l'acide carboxylique formé à partir de l'ester vinylique mis en oeuvre, et le cas échéant on traite l'halogénoacétate de vinyle séparé sous la forme de la phase organique par un agent déshydratant.

2. Procédé selon la revendication 1, caractérisé en ce que l'acide halogénoacétique mis en oeuvre est l'acide chloracétique (Cl-CH₂-COOH).

3. Procédé selon la revendication 1 ou 2 caractérisé en ce que l'ester vinylique mis en oeuvre est l'acétate de vinyle (CH₃-COO-CH=CH₂).

4. Procédé selon une des revendications 1 à 4, caractérisé en ce que le catalyseur mis en oeuvre est PdCl₂, Pd(CH₃COO)₂ ou Pd(Cl-CH₂-COO)₂ en quantité de 10 à 100 mmol/l de la phase liquide.

5. Procédé selon une des revendications 1 à 4, caractérisé en ce que le catalyseur auxiliaire mis en oeuvre est CuCl₂, Cu(CH₃-COO)₂ ou Cu(Cl-CH₂-COO)₂, à un rapport molaire de 1 : 3 à 3 : 1 avec le palladium.

6. Procédé selon une des revendications 1 à 5, caractérisé en ce que l'activateur utilisé est NaCl, CH₃-COONa ou Cl-CH₂-COONa, à un rapport molaire de 2 : 1 à 10 : 1 avec le palladium.

7. Procédé selon une des revendications 1 à 6, caractérisé en ce que l'on fait réagir un mélange ester vinylique/acide halogénoacétique à des proportions relatives de 60 : 40 à 90 : 10 parties en poids.

8. Procédé selon une des revendications 1 à 7, caractérisé en ce que l'on extrait l'halogénoacétate de vinyle brut par 1 à 10 fois son poids d'eau.

9. Procédé selon la revendication 8, caractérisé en ce que l'on réalise l'extraction en continu dans un appareil à mélangeur-décanteur ou dans une colonne.

10. Procédé selon une des revendications 1 à 7, caractérisé en ce que l'on réalise l'évaporation instantanée dans un évaporateur à couche mince.

11. Procédé selon une des revendications 1 à 10, caractérisé en ce que
a) à partir du condensat de la vaporisation instantanée à pression normale, on sépare l'ester vinylique non converti et on peut le recycler sous cette forme dans le procédé.
b) dans une distillation fractionnée sous vide, on sépare le culot de la distillation à pression normale en halogénoacétate de vinyle brut et en l'acide carboxylique formé et
c) on recycle dans le procédé l'acide halogénoacétique non converti restant en produit de culot à la distillation sous vide.

12. Procédé selon une des revendications 1 à 11, caractérisé en ce que l'on traite l'halogénoacétate de vinyle humide par un agent déshydratant consistant en sulfate de sodium, sulfate de magnésium ou chlorure de magnésium.

## Claims

1. A process for the preparation of a vinyl haloacetate of the formula (I)
HalₙHₘC-COO-CH=CH₂ (I)
in which
Hal is Cl or Br,
n is 1, 2 or 3, and
m is 3-n,
in the liquid phase in the presence of a palladium(II) compound as catalyst, a copper(II) compound as cocatalyst and a sodium compound as promoter, which comprises allowing a haloacetic acid of the formula (II)
HalₙHₘC-COOH (II)
in which Hal, n and m have the meaning given above, to react with a vinyl ester of the formula (III)
R-CH₂-COO-CH=CH₂ (III)
in which R is hydrogen or an alkyl radical having 1 to 6 carbon atoms,
at 20°C to 100°C over a period of 0.1 to 10 hours, then isolating the volatile fractions of the reaction mixture from the catalyst components in a flash evaporation,
treating the isolated catalyst components with atmospheric oxygen or hydrogen peroxide,
condensing the evaporated fractions and fractionating the condensate by distillation to give unreacted vinyl ester, crude vinyl haloacetate and unreacted haloacetic acid, extracting the crude vinyl haloacetate at 5°C to 20°C with water, as a result of which the carboxylic acid formed from the vinyl ester used is transferred into the aqueous phase, and treating the vinyl haloacetate isolated as an organic phase, if required, with a desiccant.

2. The process as claimed in claim 1, wherein the haloacetic acid used is chloroacetic acid (Cl-CH₂-COOH).

3. The process as claimed in claim 1 or 2, wherein the vinyl ester used is vinyl acetate (CH₃-COO-CH=CH₂).

4. The process as claimed in any one of claims 1 to 3, wherein the catalyst used is PdCl₂, Pd(CH₃-COO)₂ or Pd(Cl-CH₂-COO)₂ in an amount of 10 to 100 mmol/l of liquid phase.

5. The process as claimed in any one of claims 1 to 4, wherein the cocatalyst used is CuCl₂, Cu(CH₃-COO)₂ or Cu(Cl-CH₂-COO)₂ in a molar ratio of 1:3 to 3:1, based on the palladium.

6. The process as claimed in any one of claims 1 to 5, wherein the promoter used is NaCl, CH₃-COONa or Cl-CH₂-COONa in a molar ratio of 2:1 to 10:1, based on the palladium.

7. The process as claimed in any one of claims 1 to 6, wherein a vinyl ester/haloacetic acid mixture of 60:40 to 90:10 parts by weight is reacted.

8. The process as claimed in any one of claims 1 to 7, wherein the crude vinyl haloacetate is extracted with 1 to 10 times the amount of water.

9. The process as claimed in claim 8, wherein the extraction is carried out continuously in a mixer-settler apparatus or a column.

10. The process as claimed in any one of claims 1 to 7, wherein the flash evaporation is carried out in a thin film evaporator.

11. The process as claimed in any one of claims 1 to 10, wherein
a) the unreacted vinyl ester is isolated from the condensate of the flash evaporation under atmospheric pressure and can be returned to the process in this form,
b) the bottom product of the atmospheric pressure distillation is fractionated in a fractionating vacuum distillation to give crude vinyl haloacetate and the carboxylic acid formed and
c) the unreacted haloacetic acid remaining as bottom product in the vacuum distillation is returned to the process.

12. The process as claimed in any one of claims 1 to 11, wherein the water-containing vinyl haloacetate is treated with sodium sulfate, magnesium sulfate or calcium chloride as desiccant.
